(19) Europäisches Patentamt
European Patent Office
Office européen des brevets

(11) Publication number: **0 496 307 A1**

(12) **EUROPEAN PATENT APPLICATION**

(21) Application number: **92100801.7**

(51) Int. Cl.[5]: **A61K 31/40**, A61K 9/72

(22) Date of filing: **19.01.92**

(30) Priority: **24.01.91 GB 9101592**

(43) Date of publication of application:
**29.07.92 Bulletin 92/31**

(84) Designated Contracting States:
**AT BE CH DE DK ES FR GB GR IT LI LU MC NL PT SE**

(71) Applicant: **GLAXO GROUP LIMITED**
**Glaxo House, Berkeley Avenue**
**Greenford, Middlesex UB6 0NN(GB)**

(72) Inventor: **Winterborn, Ian Keith**
**Glaxo Group Research Limited, Park Road**
**Ware, Hertfordshire SG12 0DG(GB)**
Inventor: **Leak, Rosemary Elizabeth**
**Glaxo Group Research Limited, Park Road**
**Ware, Hertfordshire SG12 0DG(GB)**

(74) Representative: **Filler, Wendy Anne et al**
**Glaxo Holdings p.l.c., Glaxo House, Berkeley Avenue**
**Greenford, Middlesex UB6 0NN(GB)**

(54) **Compositions.**

(57) A pharmaceutical composition in a form adapted for intranasal administration comprising 3-[2-(dimethylamino)ethyl]-N-methyl-1H-indole-5-methanesulphonamide or a physiologically acceptable salt or solvate thereof as active ingredient in the form of a dry powder.

The intranasal compositions are of use in the treatment of conditions associated with cephalic pain, in particular migraine.

EP 0 496 307 A1

The present invention relates to a pharmaceutical composition containing as active ingredient 3-[2-(dimethylamino)ethyl]-N-methyl-1H-indole-5-methane sulphonamide, in particular a composition for intranasal administration.

3-[2-(dimethylamino)ethyl]-N-methyl-1H-indole-5-methanesulphonamide, which may here presented by the formula (I)

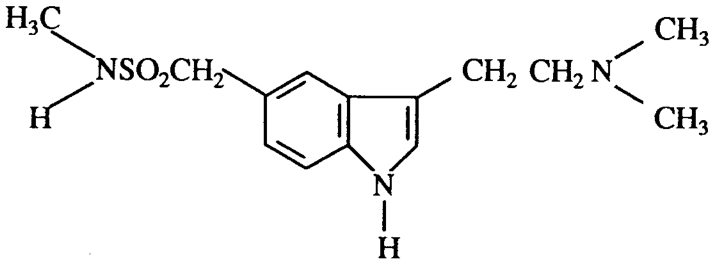

(I)

and its physiologically acceptable salts and solvates are disclosed in UK Patent Specification No. 2162522. The compound of formula (I) exhibits selective vasoconstrictor activity and is useful in the treatment of migraine.

Oral compositions have certain disadvantages for the administration of anti-migraine agents. Thus, for example, one of the symptoms associated with migraine is nausea, and the presence of nausea may make it difficult for a patient to take an oral composition. Also, it is desirable that the anti-migraine drug should be rapidly absorbed into the bloodstream. Such rapid absorption can be achieved using intranasal or parenteral administration, but some patients dislike parenteral administration, particularly if the drug is to be self-administered. Intranasal administration therefore represents a preferred route for administration of the compound of formula (I).

We have now found a particularly advantageous formulation for the intranasal administration of the compound of formula (I) comprising a dry powder.

There is thus provided according to the invention a pharmaceutical composition in a form adapted for intranasal administration comprising 3-[2-dimethylamino)ethyl]-N-methyl-1H-indole-5-methanesulphonamide or a physiologically acceptable salt or solvate thereof as active ingredient in the form of a dry powder.

For satisfactory intranasal administration, an active ingredient must be presented in a form which is readily absorbed through the nasal mucosa but which is unassociated with any adverse effects such as irritancy. Satisfactory intranasal formulations must also be sufficiently stable, chemically and physically, to be consistently dispensed in accurate metered doses, even after prolonged storage with potential temperature fluctuations of between 0 and $40^0C$. Accordingly, the active ingredient must be compatible with the excipients used in the formulation and should not aggregate in a manner which would result in a loss of accurate dose delivery, for example by caking of a powder formulation.

We have found that 3-[2-(dimethylamino)ethyl]-N-methyl-1H-indole-5-methanesulphonamide and physiologically acceptable salts and solvates thereof may advantageously be administered in the form of a dry powder.

Conveniently the compositions according to the invention will comprise a powder mix of 3-[2-(dimethylamino)ethyl]-N-methyl-1H-indole-5-methanesulphonamide or a physiologically acceptable salt or solvate thereof with at least one suitable powder carrier such as lactose, starch, starch derivatives such as hydroxypropylmethyl cellulose and polyvinylpyrrolidine (PVP). Preferably the powder carrier will form a gel in the nasal cavity, most preferably the powder carrier will be starch or modified starch, for example pregelatinised maize starch.

According to a preferred aspect the invention provides a pharmaceutical composition adapted for intranasal administration comprising a powder mix of 3-[2-(dimethylamino)ethyl]-N-methyl-1H-indole-5-methanesulphonamide or a physiologically acceptable salt or solvate thereof in a powder carrier capable of forming a gel in the nasal cavity.

According to a particularly preferred aspect the invention provides a pharmaceutical composition adapted for intranasal administration comprising a powder mix of 3-[2-(dimethylamino)ethyl]-N-methyl-1H-indole-5-methanesulphonamide or a physiologically acceptable salt or solvate thereof in a powdered starch or modified starch carrier.

Such formulations may additionally contain other excipients, for example lubricants such as sodium benzoate, flavouring agents and sweetening agents such as saccharin.

In the compositions according to the invention the compound of formula (I) or physiologically acceptable salt or solvate thereof will generally have a small particle size. As used herein small particle size means particle size of the order of 10 microns or less, preferably 2 to 5 microns. Such a particle size may be obtained by means known in the art, for example by micronisation. One or more of the components of the compositions according to the invention may be provided in the form of microspheres. Microspheres may be prepared by methods known in the art, for example by spray drying. The compositions may be prepared by intimately mixing the ingredients together, for example in a high shear mixer.

A further aspect of the invention provides a method of treating a mammal, including man, suffering from or susceptible to conditions associated with cephalic pain such as cluster headache, chronic paroxysmal hemicrania, headache associated with vascular disorders, headache associated with substances or their withdrawal (for example drug withdrawal), tension headache and in particular migraine which comprises intranasal administration of a pharmaceutical composition comprising 3-[2-(dimethylamino)ethyl]-N-methyl-1H-indole-5-methanesulphonamide or a physiologically acceptable salt or solvate thereof in the form of a dry powder. It will be appreciated that reference to treatment is intended to include prophylaxis as well as the alleviation of established symptoms.

It will be appreciated that the precise therapeutic dose of the active ingredient will depend on the age and condition of the patient and the nature of the condition to be treated and will be at the ultimate discretion of the attendant physician.

However, in general effective doses for the treatment of conditions associated with cephalic pain, for example acute treatment of migraine, will lie in the range of 0.5 to 100mg, preferably 1 to 60mg, most preferably 2 to 40mg of the active ingredient per unit dose which could be administered in single or divided doses, for example, 1 to 4 times per day.

Powder compositions are applied directly to the nasal cavity by conventional means. The formulations may be provided in single or multidose form. In the latter case a means of dose metering is provided. This may be achieved by the patient administering an appropriate, predetermined amount of the powder.

Powder compositions according to the invention may conveniently be presented in unit dose form for example in capsules or cartridges of e.g. gelatin or blister packs from which the powder may be administered by means of an inhaler. A convenient unit dose formulation for intranasal administration contains the compound of formula (I) in an amount of from 1mg to 100mg, preferably in the range of 2mg to 60mg, most preferably 2mg to 40mg.

The following non-limiting examples further illustrate the invention.

| Examples 1 and 2 | | |
|---|---|---|
| Dose: | 2mg | 40mg |
| Compound of formula (I), sulphate salt (2:1)<br>Pregelatinised Maize Starch BP | 2.33mg*<br>22.67mg | 46.6mg +<br>23.4mg |

* equivalent to 2mg free base
\+ equivalent to 40mg free base

The compound of formula (I),sulphate salt(2:1) and the pregelatinised maize starch are passed through suitable sieves and then blended together. The blend is encapsulated in hard gelatin capsules using an automatic machine. Each capsule contains the equivalent of 2mg or 40mg of compound of formula (I).

| Examples 3 and 4 | | |
|---|---|---|
| Dose: | 5mg | 10mg |
| Compound of formula I, succinate salt (1:1)<br>Lactose B.P.<br>Fill weight | 7.0mg*<br>18.0mg<br>25mg | 14.0mg +<br>36.0mg<br>50mg |

* equivalent to 5mg free base
\+ equivalent to 10mg free base

The compound of formula I, succinate salt (1:1) and the lactose were blended together and hand-filled into hard gelatin capsule shells, size 3 to give capsules containing the equivalent of 5 or 10mg of the compound of formula I.

| Examples 5 and 6 | | |
|---|---|---|
| Dose: | 5mg | 10mg |
| Compound of formula I, succinate salt (1:1)<br>Starch 1500<br>Fill weight | 7.0mg*<br>18.0mg<br>25mg | 14.0mg +<br>36.0mg<br>50mg |

* equivalent to 5mg free base
+ equivalent to 10mg free base

The compound of formula I, succinate salt (1:1) and the starch 1500 were blended together and hand-filled into hard gelatin capsule shells, size 3 to give capsules containing the equivalent of 5 or 10mg of the compound of formula I.

## Claims

**1.** A pharmaceutical composition in a form adapted for intranasal administration comprising 3-[2-(dimethylamino)ethyl]-N-methyl-1H-indole-5-methanesulphonamide or a physiologically acceptable salt or solvate thereof as active ingredient in the form of a dry powder.

**2.** A pharmaceutical composition as claimed in Claim 1 which comprises a powder mix of active ingredient with at least one pharmaceutically acceptable powder carrier or excipient.

**3.** A pharmaceutical composition as claimed in Claim 2 wherein the powder carrier is capable of forming a gel in the nasal cavity.

**4.** A pharmaceutical composition as claimed in Claim 2 or Claim 3 wherein the powder carrier is starch or modified starch.

**5.** A pharmaceutical composition as claimed in any one of Claims 1 to 4 wherein the active ingredient has a particle size of the order of 10 microns or less.

**6.** A pharmaceutical composition as claimed in any one of Claims 1 to 5 in the form of a dosage unit comprising a powder inhalation capsule or cartridge.

**7.** A pharmaceutical composition according to Claim 6 wherein the dosage unit contains 1 to 100mg of 3-[2-(dimethylamino)ethyl]-N-methyl-1H-indole-5-methanesulphonamide.

**8.** A method of treating a mammal, including man, suffering from or susceptible to cephalic pain, in particular migraine, which comprises intranasal administration of a pharmaceutical composition comprising 3-[2-(dimethylamino)ethyl]-N-methyl-1H-indole-5-methanesulphonamide or a physiologically acceptable salt or solvate thereof as active ingredient in the form of a dry powder.

European Patent Office

# PARTIAL EUROPEAN SEARCH REPORT

which under Rule 45 of the European Patent Convention shall be considered, for the purposes of subsequent proceedings, as the European search report

Application Number
EP 92 10 0801

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | FR-A-2 568 571 (GLAXO GROUP LTD) * Page 3, line 24 - page 4, line 13; page 6, lines 18-28; page 9, line 19 - page 10, line 11; page 37, line 19 - page 39, line 21 * & GB-A-2 162 522 | 1,2,5-8 | A 61 K 31/40<br>A 61 K 9/72 |
| D,Y | --- | 3,4 | |
| Y | DERWENT FILE SUPPLIER, 1988, accession no. 88-178953 [26], Derwent Publications Ltd, London, GB; & JP-A-63 115 821 (TEIJIN K.K.) 20-05-1988 * Abstract * --- | 3,4 | |
| Y | EP-A-0 122 036 (TEIJIN LTD) * Claims 1,2 * ----- | 3,4 | |

TECHNICAL FIELDS SEARCHED (Int. Cl.5)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

Remark: Although claim 8 is directed to a method of treatment of the human body (Art. 52(4) EPC) the search has been carried out and based on the alleged effects of the composition.

| Place of search | Date of completion of the search | Examiner |
|---|---|---|
| THE HAGUE | 05-03-1992 | BOULOIS D.J-M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document